# EUROPEAN PATENT SPECIFICATION

(11) **EP 2 839 801 B1**
(45) Date of publication and mention of the grant of the patent: **01.02.2017**
(21) Application number: 13184223.9
(22) Date of filing: 13.09.2013
(51) Int. Cl.: A61B 17/42, A61B 17/02, A61B 17/00

(54) **Surgical system including a support for an instrument**
Chirurgisches System mit einem Träger für ein Instrument
Système chirurgical comprenant un support pour un instrument

(30) Priority: 19.08.2013 US 201313969610
(43) Date of publication of application: 25.02.2015
(73) Proprietor: Coloplast A/S, 3050 Humlebaek (DK)
(72) Inventor: Gaynor, Allen, Coon Rapids, MN Minnesota 55448 (US)

(56) References cited:
- EP-A1- 1 972 264
- WO-A1-2009/023248
- US-A- 5 918 844
- US-A1- 2008 121 765
- US-A1- 2012 172 850
- US-A1- 2013 099 081

## Description

### Background

There is a trend to move toward minimally invasive surgical procedures that allow the patient to recover faster. Faster recoveries are associated with less time in post anesthesia and other care units, which can translate to a lower cost of patient care.

Many such minimally invasive surgical procedures are performed laparoscopically through multiple access ports formed in the abdomen. At least one access port is formed to provide access for a camera that allows visualization of the internal organs, and at least one access port is formed to provide access for surgical tools to the internal organs. However, it is often the case that the organ selected for surgical intervention will have a surface that is oriented away from the camera such that the surgeon has an imperfect view of the complete organ.

US 2012/172580 discloses a support mechanism for a medical device that includes an adjustable support having a plurality of elongate members movable relative to one another and a bracket adapted to be coupled to the adjustable support and to a first medical device.

Surgeons would welcome a new system for manipulating the orientation of internal organs to provide a better view of all surfaces of the organ.

### Summary

One aspect provides a surgical system including an instrument and a support according to claim 1 The invention is defined in claim 1. Further embodiments of the invention are defined in the dependent claims.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a perspective view of one embodiment of a surgical system including a support attached to an instrument.
Figure 2 is a perspective view of one embodiment of the instrument illustrated in Figure 1.
Figure 3 is a side view of one embodiment of a cradle of the support illustrated in Figure 1.
Figure 4 is a perspective view of the cradle illustrated in Figure 3.
Figure 5 is a top cross-sectional view of the cradle illustrated in Figure 3.
Figure 6 is a schematic view of the system illustrated in Figure 1 attached to a surgical table.
Figures 7-8 are schematic views of the system illustrated in Figure 1 in relation to a patient.
Figures 9-10 are cross-sectional views of one embodiment of a system including a support with a locking mechanism securing the support to an instrument.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Anterior means "forward" or "front," and posterior means "rearward" or "back." Relative to surfaces of an organ in the human body, an anterior surface of an instrument inserted into the organ will be oriented forward toward the belly and a posterior surface will be oriented rearward toward the spine.

Embodiments provide a surgical system including a support and an instrument that is useful in manipulating an internal pelvic organ. The surgical instrument includes a head attached to a handle, where the head is insertable into the vagina and the handle is useful in moving the head to manipulate the vagina for repair of the vaginal wall. The support is provided to hold the instrument at the location selected by the surgeon after the head of the instrument has been adjusted to manipulate a wall of the vagina. Typically, a surgical assistant hold an inserted instrument at its location within the vagina, which can lead to fatigue for the assistant. The system provides an improved surgical procedure that reduces fatigue the surgical staff and has the potential to improve surgical outcomes.

The system includes a cradle that secures the instrument to the support. The cradle is configured to allow the instrument to be removed by moving the instrument in a direction away from the patient, and advantageously, is also configured to prevent movement of the instrument in a direction closer (or further into) the patient.

The system is useful in gynecological, colorectal and other procedures. The instrument may be manually deployed into an organ during an open procedure, or the head of the instrument may be employed in a laparoscopic procedure or robotically manipulated in a robotically-assisted surgical procedure.

For example, in a laparoscopic procedure a camera system is inserted into a camera port formed through the wall of the abdomen to allow visualization of the internal organs. Other ports are formed in the abdomen to allow tools and devices to access a selected organ. The selected organ will have a surface oriented toward the camera (an anterior surface) and a surface away from the camera (a posterior surface). The head of the instrument is provided with a hinged movable plate that operates to present or displace the posterior surface of the selected organ in a direction for improved visualization by the camera. This feature is particularly useful when manipulating a posterior wall of the vagina that is typically oriented to face away from the abdomen and away from a camera that is inserted into the abdomen laparoscopically.

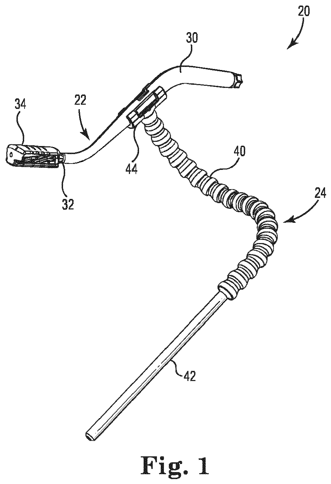

Figure 1 is a perspective view of one embodiment of a surgical system 20. The surgical system 20 (system 20) includes an instrument 22 removably attached to a support 24. The instrument 22 includes a tapered handle 30 that converges to a distal end 32 that is attached to a head 34. The support 24 includes a flexible section 40 that is connected between a post 42 that is attachable to a surgical table and a cradle 44 that is configured to removably receive the tapered handle 30 of the instrument 22.

The instrument 22 is securely held within the cradle 44 during the surgical procedure. The flexible section 40 is configured to allow the instrument to be moved to a desired location, and thereafter maintain the instrument at the desired location. The flexible section 40 is movable relative to 3 major axes to provide a pitch movement (up-and-down) for the instrument 22, a yaw movement (left and right) for the instrument 22, and a roll movement (around a longitudinal axis of the handle 30) for the instrument 22. The flexible section 40 is suitably fabricated from a set of nested components, where each nested component is movable relative to its neighbor. One such suitable flexible section is available from JOBY, San Francisco, CA.

The post 42 is sized and configured to be attached to a surgical table. In one embodiment, the post 42 is provided as a stainless steel rod or peg having a diameter of about 15.9mm (5/8 inch), although other diameter sizes are also acceptable. The post 42 may be suitably fabricated from other materials, such as plastics or resins.

During use, for example when assisting in a laparoscopic surgical procedure to repair a prolapsed vagina, the surgeon will secure the instrument 22 to the cradle 44 and insert the head 34 into the vagina. The cradle 44 holds the instrument 22 securely, but is configured to allow the instrument 22 to be released from the cradle 44. The head 34 is operable to maneuver the walls of the vagina to a desired position that orients the vagina in its natural location and allows the subsequent attachment of support material to the vagina. The support 24 is provided to hold the instrument 22 at the position selected by the surgeon, and this relieves the surgeon's assistant from the task of holding the instrument 22. The flexible section 40 provides multiple degrees of motion so as to allow the surgeon to freely maneuver the instrument 22.

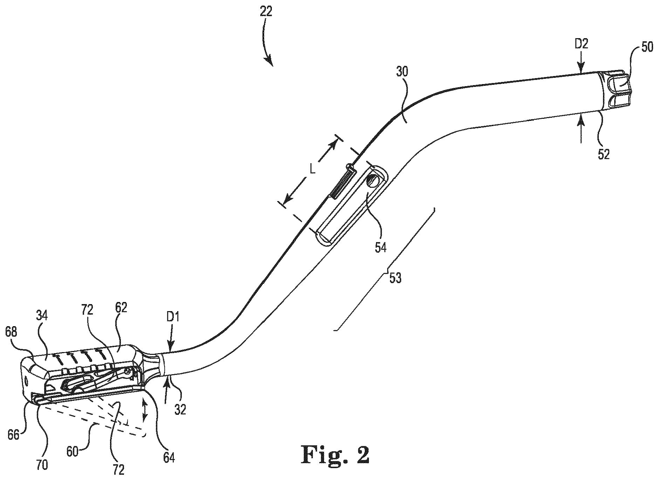

Figure 2 is a perspective view of one embodiment of the instrument 22. The instrument 22 includes a control knob 50 (or actuator 50) located at a proximal end 52 of the tapered handle 30. The control knob 50 is attached by a suitable driving mechanism to a hinged plate 60 that is attached to head 34. Rotating the control knob 50 moves the hinged plate 60 away from the head 34, and alternatively, toward the head 34.

The handle 30 is tapered to converge from the proximal end 52 toward the distal end 32. The handle 30 is wider at the proximal end 52 as compared to the distal end 32. For example, a first diameter D1 at the distal end 32 is sized to be less than a second diameter D2 at the proximal end 52. An approximate mid-portion 53 of the handle 30 is narrower than the proximal end 52 but wider than the distal end 32.

The tapered handle 30 includes a planar indentation 54 provided on each lateral side of the handle 30. The planar indentation 54 is formed as a relief that is carved out of the tapered handle 30. The indentation 54 may be molded into the handle 30 or milled out of the handle 30. The planar indentation 54 provides an engagement surface for the cradle 44 (Figure 1) to pinch or impinge against, which operates to couple the tapered handle 30 securely to the cradle 44. The planar indentation 54 has a length L that extends the longitudinal length of the relief portion that is carved from the tapered handle 30.

The handle 30 is rigid and provided so that the handle 30 does not bend as the surgeon is manipulating the organ inside the patient. In this regard, the surgeon might apply a force to the handle 30 between 1-10 pounds and the handle 30 is fabricated to be rigid to exhibit substantially zero strain in response to this level of stress applied to the handle 30 during tissue manipulation.

The head 34 includes an anterior surface 62 opposite a posterior surface 64. The hinged plate 60 has a distal side 66 (a front side) that is attached to a distal portion 68 (or a front portion) of the head 34 by a hinge 70. The hinged plate 60 is referred to as a kick out door and operates to displace the posterior wall of the vagina for better visualization during a laparoscopic procedure. In particular, the posterior surface 64 of the head 34 will be oriented toward the patient's sacrum during use, and the hinged plate 60 moves to push the posterior wall of the vagina into the surgeon's line of sight during the laparoscopic procedure.

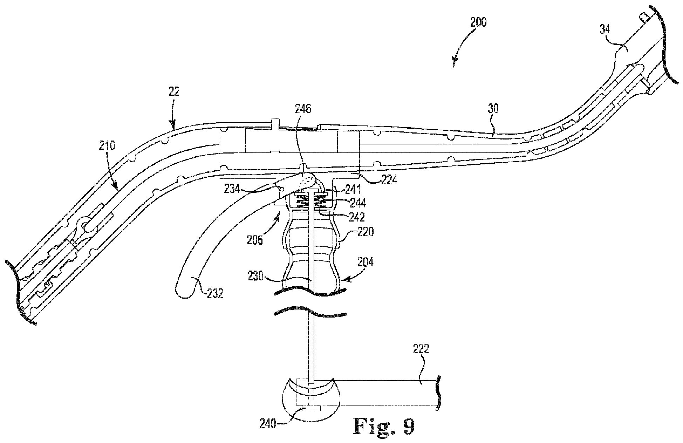

The hinged plate 60 swings or moves relative to the hinge 70. The hinge 70 attaches the distal, front side 66 of the plate 60 to the distal, front portion 68 of the posterior surface 64 of the head 34. The cantilevered motion of the door 60 relative to the head 34 is controlled by the control knob 50 at the opposite end of the instrument 22. The actuator knob 50 is connected to a movable arm 72 that is attached between the hinged plate 60 and the head 34. The rotation of the control knob 50 is converted into translational movement of the movable arm 72 by the driving mechanism (See Figure 9), which results in the hinged plate 60 moving away from (or toward) the posterior surface 64 of the head 34.

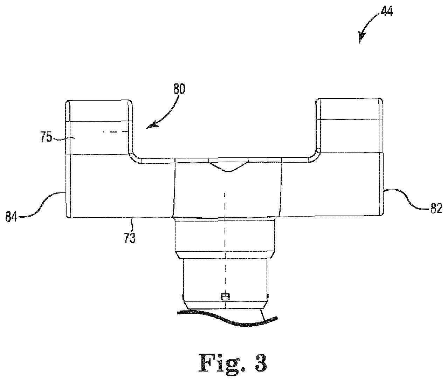

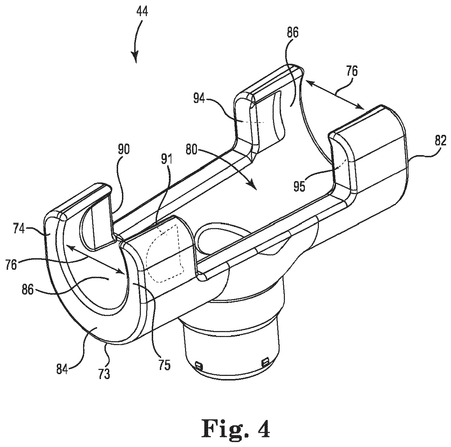

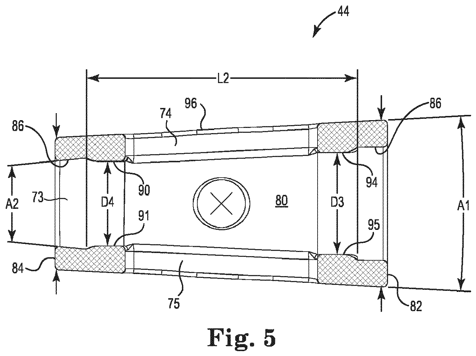

Figure 3 is a side view, Figure 4 is a perspective view, and Figure 5 is a cross-sectional view of one embodiment of the cradle 44. The cradle 44 includes a base 73 that connects with the support 24 and opposed side walls 74, 75 connected to the base 73. The opposed side walls 74, 75 extend from the base 73 and terminate at an open slot 76 formed in the cradle 44 opposite of the base73. The open slot 76 communicates with a tapered trough 80 that is formed longitudinally in the cradle 44.

The trough 80 extends from a proximal end 82 to a distal end 84 of the cradle 44. The trough 80 is tapered and converges toward the distal end 84 such that the trough 80 provides a complementary shape to receive the tapered handle 30 (Figure 2).

The converging trough 80 has an interior surface 86 that narrows from a wider dimension D3 at the proximal end 82 down to a narrower dimension D4 at the distal end 84. The interior surface 86 has a first distal pad 90 and a second distal pad 91 that each project away from an interior lateral wall of the interior surface 86. The distal pads 90, 91 are located in a front portion of the trough 80. Adjacent to the proximal end 82, the interior surface 86 has a first proximal pad 94 and a second proximal pad 95 that each project away from an interior lateral wall of the interior surface 86. The distal pads 90, 91 and the proximal pads 94, 95 each have a height projecting from the interior surface 86 that configures the tapered trough 80 to clasp the planar indentation on each lateral side of the tapered handle 30. The distal pads 90, 91 and the proximal pads 94, 95 provide an engagement feature for the cradle 44 and are adapted to engage with a respective one of the planar indentations 54 (Figure 2) that is formed in the tapered handle 30.

The trough 80 is tapered to converge toward the distal end 84. In one embodiment, the exterior surface of the cradle 44 is provided in a cylindrical shape having parallel opposed exterior walls. In one embodiment, an exterior surface 96 of the cradle 44 also converges from the proximal end 82 toward the distal end 84 as illustrated in Figure 5. The tapered and converging nature of the exterior surface 96 is represented by the angle of taper A1, where the angle of taper A1 is in a range between 2-10 degrees. In one embodiment, the angle of taper A is about 6 degrees. The tapered and converging nature of the trough 80 is represented by the angle of taper A2, where the angle of taper A2 is in a range between 7-20 degrees. In one embodiment, the angle of taper A is about 11 degrees.

The converging tapered trough 80 is fabricated to have a complementary shape to receive the tapered handle 30 of the instrument 22 (Figure 2). The tapered trough 80 is formed longitudinally in the cradle 44 from the proximal end 82 to the distal end 84, and the tapered trough 80 has taper angle A that is substantially equal to a taper angle of the tapered handle 30 at the mid-potion 53 (Figure 2) such that the tapered trough 80 is shaped to frictionally receive the tapered handle 30 of the instrument 22.

With reference to Figure 5, the pads 90, 91 are spaced a distance apart from the pads 94, 95 to configure the pads to engage with the planar indentation 54 (Figure 2) provided on the handle 30. For example, in one embodiment a distal most end of the distal pads 90, 91 is spaced a distance L2 apart from a proximal most end of the proximal pads 94, 95, and the distance L2 is selected to be approximately equal to the length L of the planar indentation 54 (Figure 2). With this configuration, movement of the tapered handle 30 in a distal direction within the trough 80 secures the instrument 22 to the cradle 44 by seating the distal pads 90, 91 to a front location of the planar indentation 54 and seating the proximal pads 94, 95 to a rear location of the planar indentation 54. The pads 90, 91 and 94, 95 are sized to frictionally engage with the planar indentation 54 such that the instrument 22 will snap-fit within the cradle 44.

The engagement feature pads 90, 91 and planar section 54 combine to lock the tapered handle 30 into the tapered trough 80 to prevent forward proximal motion of the head 34 of the instrument into the vagina. The instrument 22 is removable from the cradle 44 without having to push the instrument 22 closer to the patient. In other words, the cradle 44 is advantageously designed to allow for removal of the instrument 22 from the cradle 44 with movement in the proximal direction (toward the surgeon) that moves the instrument 22 away from the patient.

Suitable materials for fabricating the cradle 44 include metal such as stainless steel, plastics such as nylon or acrylonitrile butadiene styrene (ABS) or the like, or resins. The instrument 22 is sterilizable and configured for use as a disposable surgical instrument. Fabricating the instrument from stainless steel configures the instrument to be sterilizable and reusable.

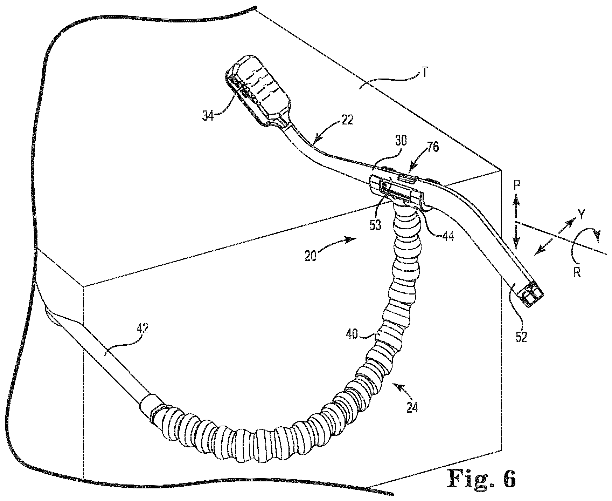

Figure 6 is a schematic view of the system 20 coupled to a surgical table T. The post 42 is secured to a holder provided that is usually provided on a surgical table T and the tapered handle 30 of the instrument 22 is secured into the cradle 44. The flexible section 40 is movable relative to the table and allows the cradle 44 to move in all of the cardinal directions in three-dimensional space including. The freedom of motion of the cradle 44 allows the instrument 22 to translate in space, to pitch P up/down on its longitudinal axis, or yaw Y left/right on its longitudinal axis, or roll R about on its longitudinal axis.

Both the proximal end 52 of the tapered handle 30 and the head 34 of the instrument 22 are wider than the widest inside width (D3) of the tapered trough 80 (Figure 5). The open slot 76 is sized to allow the mid-portion 53 of the tapered handle 30 to be inserted into the tapered trough 80 of the cradle 44. The narrower distal portion of the tapered handle 30 is inserted into the open slot 76 of the cradle 44, and the instrument 22 is pushed toward the table T until the mid-portion 53 of the tapered handle 30 is engaged or pinched or secured or locked into the cradle 44. The forward or distal movement of the instrument 22 relative to the table T operates to lock the tapered handle 30 into the cradle. The rearward or proximal movement of the instrument 22 toward the surgeon operates to unlock the instrument 22 from the cradle 44. The system 20 is adapted to allow the instrument 22 to disengage from the cradle 44 when the instrument 22 is moved rearward, for example in response to movement of the patient. In this manner, the instrument 22 is adapted to release and give way so that undue force or discomfort is not delivered to the patient.

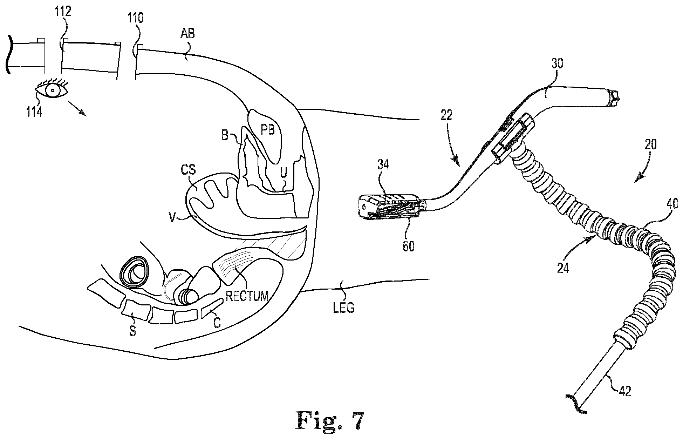

Figures 7 - 8 are schematic views of the system 20 employed to internally manipulate an orientation of the vagina V of the patient during a laparoscopic procedure. Figures 7 - 8 represent the related anatomy but are not drawn to scale. The laparoscopic procedure may be of the robotically-assisted type of laparoscopic procedure. The instrument 22 is suited for manual use in dissecting tissues off of the vagina V and in manipulating the orientation of the vagina V. Although features of a laparoscopic vaginal procedure are described below, it is to be understood that the system 20 is suitable for manually manipulating the vagina or other pelvic organs in other surgical procedures, including other robotic procedures and the like.

Figures 7-8 are schematic views of internal organs of a supine patient with the head 34 of the instrument 22 in position for insertion into the vagina V. A natural vagina has an entrance and terminates at the cervix, which communicates with the uterus. Some women have their uteruses removed through a hysterectomy, and some of these procedures result in the presence of a cervical stump CS connected to the vagina V as illustrated. The bladder B communicates with the urethra U and is located anterior to the vagina V and posterior to the pubic bone PB. The digestive tract and the rectum are located posterior to the vagina V. The sacrum S and the coccyx C are located posterior to the digestive tract (rectum). The abdominal wall AB protects and supports the internal organs.

During a laparoscopic surgical procedure, one or more access ports are formed through the abdominal wall AB (usually supported by a trocar) to allow a visualization camera and tools to access the internal organs. In the illustrated embodiment, a first trocar 110 provides an access port for surgical tools and a second trocar 112 provides an access port for an optical camera 114. One or more additional ports (for example a nitrogen inflation port) may be provided through the abdominal wall AB in what is traditionally described as a trans-abdominal approach to the vagina V.

The system 20 is secured to the table T and is used to dissect vesico-vaginal tissue away from a wall of the vagina V. One or more surgical tools are inserted through the trocar 110 toward the vagina V. The surgeon moves the head 34 of the instrument 22 to a desired location to position the vagina V for surgery and the support 24 holds the instrument 22 in the selected location. The head 34 is sized to manipulate the vagina V and to displace portions of the wall of the vagina, which allows the surgeon to progressively dissect the vesico-vaginal tissue 122 between the bladder B and the anterior wall 120 of the vagina V. It is desirable to expose the anterior wall 120 of the vagina V to allow the surgeon to optimally orient the vagina V when addressing prolapse and in improving support provided to the vagina V, for example during a sacrocolpopexy procedure.

The plate 60 of the head 34 is movable to dissect recto-vaginal tissue away from a wall of the vagina V. The surgeon employs the handle 30 to provide a slight lifting force to the vagina V and the support 24 maintains this force and the location of the instrument 22. Suitable other tools are employed to dissect the recto-vaginal tissue from between a posterior wall 124 of the vagina V and a sheath or other tissue layers attached to the rectum. Although not shown, the instrument 22 is also useful for manipulating the vagina V to allow the surgeon to relieve the uterosacral ligament and to access and relieve other connective tissues attached between the vagina V and other organs.

After appropriate dissection the anterior wall 120 and the posterior wall 124 of the vagina V will be separated from the bladder/rectal connective tissue, respectively. Organs and tissue inside of the abdomen can obstruct the surgeon's view of the vagina V. The head 34 of the instrument 22 advantageously provides a backboard or surface that supports the anterior wall 120 of the vagina to allow the surgeon to suture or otherwise surgically intervene in repairing the vagina V.

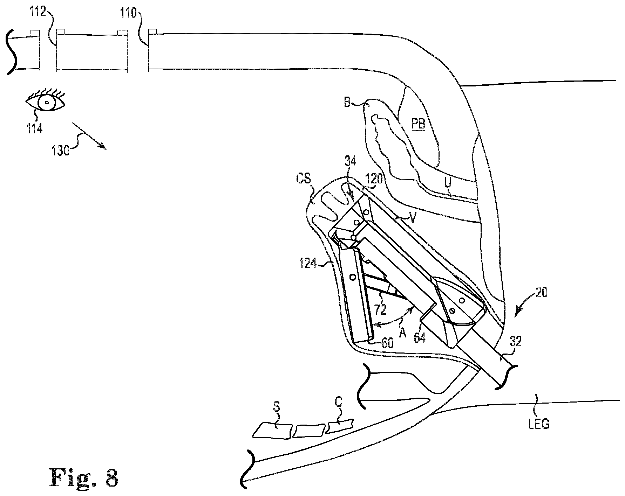

Figure 8 is a schematic view of the vagina after the anterior wall 120 and the posterior wall 124 of the vagina V have been separated from the bladder/rectal connective tissue, respectively.

The posterior wall 124 of the vagina V, and in particular, the distal posterior wall of the vagina V in the direction of the vaginal opening, is typically impeded by other tissues and hidden from the view of the surgeon during laparoscopic surgery. The hinged plate 60 has been pivoted away from the posterior surface 64 of the head 34 to move (or "kick out") the posterior wall 124 of the vagina V into the line of sight 130 of the camera 114 that is positioned trans-abdominally.

Figures 9-10 are cross-sectional views of one embodiment of a system 200. The system 200 includes the instrument 22 and a support 204 with a locking mechanism 206 securing the support 204 to the handle 30 of the instrument 22.

Figure 9 illustrates the locking mechanism 206 engaged in a locked state that operates to lock the support 204 in place.

The instrument 22 is as described above and includes the tapered handle extending between the control knob 50 and the head 34. The driving mechanism 210 is connected between the control knob 50 and the hinged plate 60 and operates to translate the rotational movement of the control knob 50 (Figure 2) into movement of the hinged plate 60.

The support 204 includes a flexible section 220 that extends between a rod 222 that is attachable to a surgical table and a cradle 224 that receives the tapered handle 30 of the instrument 22. The dimensions of the cradle 224 are similar to the cradle 44 described above. In this regard, the cradle 224 is a tapered configuration that is complementary to the tapered shape of the handle 30.

The locking mechanism 206 includes a keeper 230 connected between the rod 222 and the cradle 224, and a release lever 232 that is secured to a lower portion of the cradle 224 by a pivot 234. The keeper 230 is one of a flexible rod or a braided cable. The keeper 230 projects through the rod 222 to a base flange 240 on one end and extends to an opposite end that is attached to flanges 241 and 242. The flange 241 is a movable flange and the flange 242 is a fixed flange that is fixed to the walls of the flexible section 220. The movable flange 241 is biased relative to the fixed flange 242 by a spring 244.

The locking mechanism 206 is provided as a spring applied pressure release locking mechanism. The locking mechanism 206 is illustrated in Figure 9 in the closed or locked state with the lever 232 in a down position. In the closed or locked state the flexible section 220 is maintained in a rigidly stiff configuration that locks or holds the support 204 in a position selected by the surgical staff. The handle 232 pivots about pivot 234 and the spring 244 forces the movable flange 241 upwards toward the cradle 224 to create tension in the keeper 230. In this manner, the flexible section 220 is placed under tension between the rod 222 and the cradle 224, which locks the segments of the flexible section 220 and reduces its flexibility.

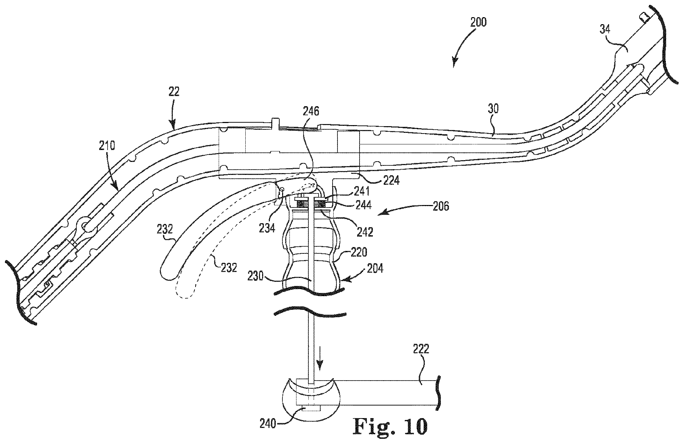

Figure 10 illustrates the locking mechanism 206 in an open or unlocked position. The lever 232 has been moved to the up position which moves the movable flange 241 and the keeper 230 downward, which allows the keeper 230 to become slackened. The slack in the keeper 230 allows the segments of the flexible section 220 to move relative to each other, which allows the surgical staff to selectively position the instrument 22 held in the cradle 224.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention be limited only by the claims and the equivalents thereof.
toward the cradle 224 to create tension in the keeper 230. In this manner, the flexible section 220 is placed under tension between the rod 222 and the cradle 224, which locks the segments of the flexible section 220 and reduces its flexibility.

Figure 10 illustrates the locking mechanism 206 in an open or unlocked position. The lever 232 has been moved to the up position which moves the movable flange 241 and the keeper 230 downward, which allows the keeper 230 to become slackened. The slack in the keeper 230 allows the segments of the flexible section 220 to move relative to each other, which allows the surgical staff to selectively position the instrument 22 held in the cradle 224.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention be limited only by the claims and the equivalents thereof.

## Claims

1. A surgical system (20) comprising:
an instrument (22) having a tapered handle (30) attached to a head (34), with the head (34) sized for insertion into a vagina and the tapered handle (30) having a distal end (32) that is narrower than a proximal end (52) of the tapered handle (30);
a support (24) including a flexible section (40) that is connected between a post (42) that is attachable to a surgical table and a cradle (44) that is configured to removably receive the tapered handle (30) of the instrument (22);
wherein the cradle (44) includes a base (73) connected to the support (24) and opposed side walls (74, 75) connected to the base (73), the opposed side walls (74, 75) extend from the base (73) and terminate at an open slot (76) formed in the cradle (44) opposite of the base (73), the open slot (76) communicates with a tapered trough (80) that is formed longitudinally in the cradle (44) from a proximal end (82) to a distal end (84) of the cradle (44);
wherein the tapered trough (80) has a first inside width between the opposed side walls (74, 75) at the distal end (84) of the cradle (44) that is less than a second inside width between the opposed side walls (74, 75) at the proximal end (82) of the cradle (44) such that the tapered trough (80) is sized to receive the tapered handle (30) of the instrument (22);
wherein the cradle (44) includes an engagement feature that operates to pinch the tapered handle (30) between the opposed side walls (74, 75) of the tapered trough (80) when the tapered handle (30) of the instrument (22) is moved in a distal direction within the tapered trough (80) **characterised in that**
the tapered handle (30) includes a planar indentation (54), and the engagement feature of the cradle (44) is a projection formed on one of the opposed side walls (74, 75) of the cradle (44), the projection is sized to impinge against the planar indentation (54) formed in the tapered handle (30).

2. The surgical system of claim 1, wherein the head (34) has an anterior surface (62) opposite a posterior surface (64) and the posterior surface (64) includes a hinged plate (60), the hinged plate (60) is attached to a distal portion (68) of the posterior surface (64) of the head (34) by a hinge (70).

3. The surgical system of claim 2, wherein the distal end (32) of the tapered handle (30) is attached to the head (34) and the proximal end (52) of the tapered handle (30) includes an actuator (50) that is attached to the hinged plate (60).

4. The surgical system of claim 1, wherein the open slot (76) extends from the proximal end (82) to the distal end (84) of the cradle (44) and is so configured such that movement of the tapered handle (30) of the instrument (22) in a proximal direction within the tapered trough (80) allows the instrument (22) to be passed through the open slot (76) and removed from the cradle (44).

5. The surgical system of claim 1, wherein the tapered handle (30) is rigid and so configured to exhibit substantially zero strain in response to a stress applied to the handle during tissue manipulation.

6. The surgical system of claim 1, wherein the flexible section (40) is movable to allow the cradle (44) to pitch up/down on its longitudinal axis, yaw left/right on its longitudinal axis, and roll about on its longitudinal axis.

7. The surgical system of claim 1, wherein the support (204) includes a locking mechanism (206) having a keeper (230) connected between the post (222) and the cradle (224) and a spring (244) configured to bias the locking mechanism (206) into an open state that allows the flexible section (220) to flex.

8. The surgical system of claim 11, further comprising:
a lever (232) attached to the cradle (224) by a pivot point (234), the lever (232) movable relative to the pivot point (234) to compress the spring (244) to lock the locking mechanism (206) and secure the flexible section (220) in a locked state.

## Patentansprüche

1. Chirurgisches System (20), umfassend:
ein Instrument (22) mit einem verjüngten Griff (30), der an einem Kopf (34) befestigt ist, wobei der Kopf (34) eine Größe zur Einführung in eine Vagina aufweist, und der verjüngte Griff (30) ein distales Ende (32) aufweist, das schmäler als ein proximales Ende (52) des verjüngten Griffs (30) ist;
einen Träger (24) mit einem flexiblen Abschnitt (40), der zwischen einem Stab (42), der an einem Operationstisch befestigt werden kann, und einer Haltevorrichtung (44), die dazu ausgelegt ist, den verjüngten Griff (30) des Instruments (22) entfernbar aufzunehmen, angekoppelt ist;
wobei die Haltevorrichtung (44) eine Basis (73), die mit dem Träger (24) verbunden ist, und gegenüberliegende Seitenwände (74, 75), die mit der Basis (73) verbunden sind, aufweist, wobei sich die gegenüberliegenden Seitenwände (74, 75) von der Basis (73) erstrecken und an einem offenen Schlitz (76) enden, der in der Haltevorrichtung (44) gegenüber der Basis (73) ausgebildet ist, wobei der offene Schlitz (76) mit einer verjüngten Mulde (80) in Verbindung steht, die längs in der Haltevorrichtung (44) von einem proximalen Ende (82) zu einem distalen Ende (84) der Haltevorrichtung (44) ausgebildet ist;
wobei die verjüngte Mulde (80) eine erste innere Breite zwischen den gegenüberliegenden Seitenwänden (74, 75) am distalen Ende (84) der Haltevorrichtung (44) aufweist, die kleiner als eine zweite innere Breite zwischen den gegenüberliegenden Seitenwänden (75, 75) am proximalen Ende (82) der Haltevorrichtung (44) ist, so dass die verjüngte Mulde (80) eine Größe zur Aufnahme des verjüngten Griffs (30) des Instruments (22) aufweist;
wobei die Haltevorrichtung (44) ein Eingriffsmerkmal aufweist, das den verjüngten Griff (30) zwischen den gegenüberliegenden Seitenwänden (74, 75) der verjüngten Mulde (80) einklemmt, wenn der verjüngte Griff (30) des Instruments (22) in eine distale Richtung in der verjüngten Mulde (80) bewegt wird, **dadurch gekennzeichnet, dass** der verjüngte Griff (30) eine ebenflächige Vertiefung (54) aufweist und das Eingriffsmerkmal der Haltevorrichtung (44) ein Vorsprung ist, der auf einer der gegenüberliegenden Seitenwände (74, 75) der Haltevorrichtung (44) geformt ist, wobei der Vorsprung eine solche Größe aufweist, dass er gegen die ebenflächige, in dem verjüngten Griff (30) geformte Vertiefung (54) auftrifft.

2. Chirurgisches System nach Anspruch 1, wobei der Kopf (34) eine vordere Fläche (62) gegenüber einer hinteren Fläche (64) aufweist und die hintere Fläche (64) eine Scharnierplatte (60) aufweist, wobei die Scharnierplatte (60) über ein Scharnier (70) an einem distalen Abschnitt (68) der hinteren Fläche (64) des Kopfes (34) befestigt ist.

3. Chirurgisches System nach Anspruch 2, wobei das distale Ende (32) des verjüngten Griffs (30) am Kopf (34) befestigt ist und das proximale Ende (52) des verjüngten Griffs (30) ein Stellglied (50) aufweist, das an der Scharnierplatte (60) befestigt ist.

4. Chirurgisches System nach Anspruch 1, wobei sich der offene Schlitz (76) vom proximalen Ende (82) zum distalen Ende (84) der Haltevorrichtung (44) erstreckt und so ausgelegt ist, dass eine Bewegung des verjüngten Griffs (30) des Instruments (22) in eine proximale Richtung in der verjüngten Mulde (80) es dem Instrument (22) gestattet, durch den offenen Schlitz (76) geführt und aus der Haltevorrichtung (44) entfernt zu werden.

5. Chirurgisches System nach Anspruch 1, wobei der verjüngte Griff (30) starr und so ausgelegt ist, dass er als Reaktion auf eine auf den Griff während einer Gewebemanipulation aufgebrachte Belastung im Wesentlichen Null Dehnung zeigt.

6. Chirurgisches System nach Anspruch 1, wobei der flexible Abschnitt (40) bewegbar ist, damit sich die Haltevorrichtung (44) auf ihrer Längsachse nach oben/unten bewegen kann, auf ihrer Längsachse nach links/rechts ausscheren kann und um ihre Längsachse rollen kann.

7. Chirurgisches System nach Anspruch 1, wobei der Träger (204) einen Arretiermechanismus (206) mit einem Schließer (230), der zwischen dem Stab (222) und der Haltevorrichtung (224) angekoppelt ist, und einer Feder (244), die zur Vorspannung des Arretiermechanismus (206) in einen offenen Zustand ausgelegt ist, in dem sich der flexible Abschnitt (220) biegen kann, aufweist.

8. Chirurgisches System nach Anspruch 11, ferner umfassend:
einen Hebel (232), der über einen Drehpunkt (234) an der Haltevorrichtung (224) befestigt ist, wobei der Hebel (232) bezüglich des Drehpunkts (234) bewegbar ist, um die Feder (244) zur Arretierung des Arretiermechanismus (206) und zur Fixierung des flexiblen Abschnitts (220) in einem arretierten Zustand zusammenzudrücken.

## Revendications

1. Système (20) chirurgical comportant :
un instrument (22) présentant une poignée (30) à section décroissante fixée à une tête (34), la tête (34) étant dimensionnée pour être insérée dans un vagin et la poignée (30) à section décroissante présentant une extrémité (32) distale qui est plus étroite qu'une extrémité (52) proximale de la poignée (30) à section décroissante ;
un support (24) comprenant une section (40) souple qui est reliée entre un montant (42) qui peut être fixé à une table chirurgicale et un berceau (44) qui est configuré pour recevoir de manière amovible la poignée (30) à section décroissante de l'instrument (22) ;
dans lequel le berceau (44) comprend une base (73) reliée au support (24) et des parois (74, 75) latérales opposées reliées à la base (73), les parois (74, 75) latérales opposées s'étendent depuis la base (73) et se terminent au niveau d'une fente (76) ouverte formée dans le berceau (44) à l'opposé de la base (73), la fente (76) ouverte communique avec un chenal (80) à section décroissante qui est formé longitudinalement dans le berceau (44) d'une extrémité (82) proximale à une extrémité (84) distale du berceau (44) ;
dans lequel le chenal (80) à section décroissante présente une première largeur intérieure entre les parois (74, 75) latérales opposées au niveau de l'extrémité (84) distale du berceau (44) qui est inférieure à une seconde largeur intérieure entre les parois (74, 75) latérales opposées au niveau de l'extrémité (82) proximale du berceau (44) de telle sorte que le chenal (80) à section décroissante soit dimensionné pour recevoir la poignée (30) à section décroissante de l'instrument (22) ;
dans lequel le berceau (44) comprend une caractéristique de mise en prise qui fonctionne de façon à pincer la poignée (30) à section décroissante entre les parois (74, 75) latérales opposées du chenal (80) à section décroissante lorsque la poignée (30) à section décroissante de l'instrument (22) est déplacée dans une direction distale à l'intérieur du chenal (80) à section décroissante, **caractérisé en ce que**
la poignée (30) à section décroissante comprend une indentation (54) plane, et la caractéristique de mise en prise du berceau (44) est une saillie formée sur l'une des parois (74, 75) latérales opposées du berceau (44), la saillie est dimensionnée pour buter contre l'indentation (54) plane formée dans la poignée (30) à section décroissante.

2. Système chirurgical selon la revendication 1, dans lequel la tête (34) présente une surface (62) antérieure opposée à une surface (64) postérieure et la surface (64) postérieure comprend une plaque (60) à charnière, la plaque (60) à charnière est fixée à une partie (68) distale de la surface (64) postérieure de la tête (34) par une charnière (70).

3. Système chirurgical selon la revendication 2, dans lequel l'extrémité (32) distale de la poignée (30) à section décroissante est fixée à la tête (34) et l'extrémité (52) proximale de la poignée (30) comprend un actionneur (50) qui est fixé à la plaque (60) à charnière.

4. Système chirurgical selon la revendication 1, dans lequel la fente (76) ouverte s'étend de l'extrémité (82) proximale à l'extrémité (84) distale du berceau (44) et est configurée de telle sorte que le déplacement de la poignée (30) à section décroissante de l'instrument (22) dans une direction proximale à l'intérieur du chenal (80) à section décroissante permette de faire passer l'instrument (22) dans la fente (76) ouverte et de l'enlever du berceau (44).

5. Système chirurgical selon la revendication 1, dans lequel la poignée (30) à section décroissante est rigide et configurée de telle sorte qu'elle présente une contrainte sensiblement de zéro en réponse à une force appliquée sur la poignée au cours de la manipulation de tissus.

6. Système chirurgical selon la revendication 1, dans lequel la section (40) souple est mobile pour permettre au berceau (44) d'effectuer un mouvement de tangage vers le haut/bas sur son axe longitudinal, de lacet vers la gauche/droite sur son axe longitudinal et de roulis autour de son axe longitudinal.

7. Système chirurgical selon la revendication 1, dans lequel le support (204) comprend un mécanisme (206) de verrouillage muni d'un loquet (230) relié entre le montant (222) et le berceau (224) et un ressort (244) configuré pour solliciter le mécanisme (206) de verrouillage dans un état ouvert qui permet à la section (220) souple de se plier.

8. Système chirurgical selon la revendication 11, comportant en outre :
un levier (232) fixé au berceau (224) par un point (234) de pivotement, le levier (232) étant mobile par rapport au point (234) de pivotement pour comprimer le ressort (244) afin de verrouiller le mécanisme (206) de verrouillage et d'immobiliser la section (220) souple dans un état verrouillé.
